# EUROPEAN PATENT APPLICATION

(11) **EP 1 240 890 A1**
(43) Date of publication of application: **18.09.2002**
(21) Application number: 00983489.6
(22) Date of filing: 19.12.2000
(51) Int. Cl.: A61K 7/13

(54) **HAIR DYE COMPOSITION**

(30) Priority: 20.12.1999 JP 36031399; 20.12.1999 JP 36079799
(71) Applicant: Lion Corporation, Tokyo 130-8644 (JP)
(72) Inventor: NOGUCHI, Mutsumi, Tokyo 130-8644 (JP); ONUKI, Takeshi, Tokyo 130-8644 (JP); MITAMURA, Joji, Tokyo 130-8644 (JP)
(74) Representative: Stuart, Ian Alexander
(86) International application number: JP0008987
(87) International publication number: WO01045656

(57) **Abstract**

A one-pack aerosol type hair dye composition which comprises an oxidation color and an oxidase, characterized in that at least one water-soluble high-molecular compound selected from hydroxypropyl cellulose, carboxymethyl cellulose, xanthan gum, gum guaiac, locust bean gum, gum arabic, tragacanth gum, karaya gum, gellan gum, pectin, carrageenan, furcellaran, alginic acid and salts thereof, hyaluronic acid and salts thereof, chondroitin sulfuric acid and salts thereof, ethylene oxide polymer, polyacrylic acid and salts thereof, acrylic copolymers and salts thereof, polyvinyl pyrrolidone, vinyl pyrrolidone copolymers, polyvinyl acetate, vinyl acetate copolymers, carboxy vinyl polymer is formulated.

## Description

### TECHNICAL FIELD

This invention relates to a hair dye composition of a one-pack (one-component) aerosol type, and more particularly, to an oxidation-type hair dye composition of the one-pack aerosol type wherein a hair dye composition sprayed from an aerosol container exhibits good foam retention without dripping of liquid in use, has good stability with time and shows an excellent dyeing effect after long-time storage, and can be improved in foaming property.

### TECHNICAL BACKGROUND

The oxidation-type hair dye is one which is used to dye white hair usually by mixing for reaction between an oxidation color (first agent) and an oxidizing agent (second agent) on use and acting the mixture on hair. Although these hair dyes may take a liquid, powdery or paste form in use, users should employ them in every time after mixing the first agent with the second agent for all the types thereof, so that the way to use them is troublesome and thus, it has been required to overcome the trouble from the standpoint of the usability.

In recent years, taking the usability into consideration, a one-pack aerosol type hair dye composition has been proposed. With a hair dye, if a liquid is dripped upon application to hair or after the application, a problem rises in that clothes or a floor is stained therewith. To avoid this, with a one-pack aerosol type product, it is desirable that liquid dripping be unlikely to occur upon spraying from a container.

With the case of such a two-pack type oxidation dye as mentioned above, the first agent contains an oxidation color such as para-phenylenediamine, para-aminophenol or the like, with its liquid nature being alkaline. This has presented the problem that head skin is irritated by the attack of the alkali.

Further, a hair dye using an oxidation color shows a hair dyeing effect, as stated hereinabove, through oxidation polymerization of the oxidation color in hair. The oxidation color commences the polymerization reaction outside hair in the presence of oxygen in air. In order to suppress the influence of air oxidation in the course of manufacture, it is necessary to add various types of antioxidants and reducing agents. However, it has been indicated that these additives not only inhibit oxidation, but also have the possibility of decomposing an oxidation color per se for some types of oxidation colors.

On the other hand, hydrogen peroxide has been in main use as an oxidizing agent. However, it is known that hydrogen peroxide gives a damage on hair. It is also known that a long-term use of hydrogen peroxide damages hair and brings about the adverse influence that a hair color is changed to reddish brown after hair dyeing.

Up to now, studies have been made on the neutralization of liquid nature for the first agent (Japanese Patent Laid-open No. Hei 8-217652), and an attempt to mitigate a damage on hair caused by hydrogen peroxide is proposed as a technique wherein an oxidase is used in place of hydrogen peroxide.
Instances of such techniques include the use of peroxidase as an oxidase (Japanese Patent Laid-open No. Sho 47-10400 and Japanese Patent Laid-open No. Sho 53-32132), the use of laccase (United States Patent No. 3251742 and Japanese Patent Laid-open No. Hei 6-172145), the use of uricase (Japanese Patent Laid-open No. Sho 63-246313), and the like.

However, these oxidases have the property of being unstable during storage, and where a one-pack type hair dye composition, particularly, a one-pack aerosol type composition, is prepared by mixing with an oxidation color, the oxidase reacts with the oxidation color in the composition thereby forming an insoluble coagulum. As a result, the action of the oxidase is not satisfactorily shown on use, and the formation of such an insoluble coagulum in a product presents a great problem upon use as a hair dye. Accordingly, a serious problem has been involved in that unless the insoluble matter is suppressed from being formed, the hair dye cannot be put on the market as a product, particularly as an aerosol product. Moreover, any proposal has never been found wherein usability upon dyeing of hair is taken into account, and particularly, it presents a great problem that clothes or a floor is stained by dripping of liquid on the way of application of a hair dye, so that an immediate solution therefor has been asked for.

It will be noted that for a conventional technique of improving stability of an oxidase during storage, those techniques against catalase (Japanese Patent Laid-open No. Hei 8-175935) and also against uricase (Japanese Patent Laid-open No. Hei 8-217652) are disclosed. In the disclosed techniques, it is essential to add a reducing agent to a composition, with the apprehension that the action of an oxidase is weakened by the inhibiting action of the reducing agent. In addition, for a technique of preparations, the use of enzyme-containing hair dyes and anionic surfactants is disclosed (WO9915137, WO9936046, WO9856335, and Japanese Patent Laid-open No. Hei 10-364200). However, some of the preparations have presented the problems including not only such unsatisfactory stabilization in storage as mentioned before, but also the inconvenience in use caused by an inappropriate foaming effect of an anionic surfactant.

### DISCLOSURE OF THE INVENTION

Under these circumstances in the art, the invention relates to a one-pack aerosol type oxidation hair dye which is able to save a trouble of mixing first and second agents whenever used and is thus excellent in utility, and has for its object the provision of a one-pack aerosol type oxidation hair dye composition which is improved in foaming property when sprayed from a container, is improved in usability such as by preventing a liquid from dripping upon use with good stability with time, shows an excellent hair-dyeing effect after storage over a long time, and is improved in foaming property.

We have made intensive studies in order to achieve the above object and, as a result, found that for the preparation of a hair dye composition of a one-pack aerosol type that permits hair to be dyed by filling a stock solution, which is obtained by dissolving an oxidase and an oxidation color in water, and an aerosol propellant in an aerosol container, and merely applying over hair on the whole after the liquid content (stock solution) is sprayed against a brush to be used or is sprayed directly on hair, and is thus very excellent in usability, a specific type of water-soluble, high-molecular compound is used, so that good foaming properties are attained upon spraying from the aerosol container, no dripping of liquid takes place when the composition is sprayed or applied to hair, and stability with time is good and an excellent dyeing effect is shown after storage over a long time. Further, as a result of intensive studies, it has been found that when a ratio by weight between the above-mentioned stock solution and the aerosol propellant is determined within a specified range and a gas pressure of filling the aerosol propellant in the aerosol container is set within a specific range, spraying properties are improved with more excellent foaming properties being obtained. As a result of still further intensive studies, it has been revealed that when the viscosity of the composition is controlled within a predetermined range and the pH is controlled within a predetermined range, the liquid dripping upon application to hair can be more effectively prevented with good dyeing properties. In addition, it has been also found that the formulation of a specific type of anionic surfactant leads to more excellent foaming properties. The invention has been accomplished based on these findings.

More particularly, the invention provides a one-pack aerosol type hair dye composition comprising an oxidation color and an oxidase, characterized in that at least one water-soluble, high-molecular compound selected from hydroxypropyl cellulose, carboxymethyl cellulose, xanthan gum, gum guaiac, locust bean gum, gum arabic, tragacanth gum, karaya gum, gellan gum, pectin, carrageenan, furcellaran, alginic acid and salts thereof, hyaluronic acid and salts thereof, chondroitin sulfuric acid and salts thereof, ethylene oxide polymer, polyacrylic acid and salts thereof, acrylic copolymers and salts thereof, polyvinyl pyrrolidone, vinyl pyrrolidone copolymers, polyvinyl acetate, vinyl acetate copolymers, carboxy vinyl polymer is further formulated.

Preferably, the composition should preferably have a pH of 5 to 8.5 and a viscosity of 5 to 30,000 mPa·s at 25°C. In addition, it is also preferred that an amido-type surfactant represented by the following general formula (1) and having a carboxyl group or salt thereof at a terminal thereof is contained 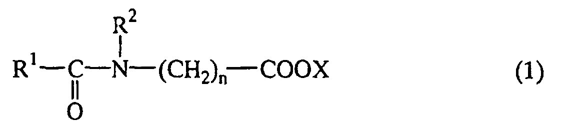 (wherein R¹ represents a linear or branched alkyl group or alkenyl group having 8 to 30 carbon atoms, R² represents a hydrogen atom or a linear or branched alkyl group or alkenyl group having 1 to 8 carbon atoms, n is 2 to 7, and X represents a hydrogen atom, an ammonium group, a sodium atom, a potassium atom or an organic amine residue).

Preferably, the ratio by weight of the stock solution and the aerosol propellant in the one-pack aerosol type hair dye composition is such that stock solution: aerosol propellant = 95:5 to 85:15, and the gas pressure of the aerosol propellant is not lower than 2 kg/cm².

The invention is described in detail below. The hair dye composition of the invention makes use of an oxidase as an oxidizing agent. The oxidase used in the invention includes laccase (E. C. 1.10.3.2), catechol oxidase (E. C. 1.10.3.1), billirubin oxidase (E. C. 1.3.3.5), monophenol monooxidase (E. C. 1. 14. 99. 1) and the like.

The laccase is an enzyme which contains a plurality of copper atoms capable of catalytically oxidizing phenol or an aromatic amine compound. As a result of the oxidation reaction with laccase, allyl oxy radicals are produced from an appropriate phenolic compound, and a dimer, an oligomer or a polymer is provided through polymerization reaction of the resultant product. The laccase is derived from a microorganism, e.g. a mycelium, a bacterium or a plant and is preferably derived from a mycelium, and is more preferably derived from Polyporus sp., particularly, P. pinsitus or P. versiocolor, Myceliphothora sp., particularly, M. thermophila, Phizocutonia sp., particularly, Rh. Practicola or Rh. solani, Pyriculania sp., particularly, P. oryzae, Scytalidium sp., such as Scytalidum thermophilium, and Rhus sp., for plant-derived one, favorably Rhus vernicifera.

Especially, with respect to oxidation-reduction enzymes, laccases belonging to Polyporus sp., particularly, those of WO 96/00290 (NOVO Nordisk Biotec Inc.) for Polyporus pinisitus-derived laccase (which may also be called Trametes Villosa-derived laccase) and those of WO 95/33836 (NOVO Nordisk Biotec Inc.) for Mytheliophthora thermophila-derived laccase are known. Moreover, with respect to Scytalidium sp.-derived laccases, particularly, S. thermophilium-derived laccase, WO 95/33837 and WO 97/19998 (NOVO Nordisk Biotec Inc.) are known, of which there are included Pyriculania sp. (Pyricularia oryzae) derived laccase that is commercially available from SIGMA Co., Ltd., the designation of No. L5510, Coprinus sp. (C. cinereus) derived laccase, and Rhizoctonia sp. (Rh. solani) derived laccase whose optimum pH ranges 6.0 to 8.5 as described in WO 95/07988.

Laccases derived from other types of bacilli are known including Collybia, Fomes, Lentinum, Pleurotus, Aspergillus, Neurospora, Podospora, and phlebia (P. radiata) as described in WO 92/01046, Coriolus sp. (C. hirsitus) in JP 2-238885, and Botrytis.

As bilirubin oxidase, Myrothecim sp. (M. verrucaria) derived one is preferred. H₂O₂ producing oxidase is usually employed along with a peroxidase capable of decomposing H₂O₂ or reducing H₂O₂ production. Examples of such peroxidase include glucose oxidase (E. C. 1. 1. 3. 4), hexose oxidase (E. C. 1. 1. 3. 5), L-amino acid oxidase (E. C. 1. 4. 3. 2), xylitol oxidase, galactose oxidase (E. C. 1. 1. 3. 9), pyranose oxidase (E. C. 1. 1. 3. 10), alcohol oxidase (E. C. 1. 1. 3. 13) and the like.

Especially, when L-amino acid oxidase is used, it should preferably be derived from Trichoderma sp., particularly, T. harzianum (WO 94/25574, NOVO Nordisk A/S) or T.viride. For glucose oxidase, Aspergillus sp. (A. niger) or Cladosporium sp. Particularly, C.oxysporum, is preferred. Hexose oxidase is derived from red alga, namely Chondrus crispus (which is recently known as Irish moss: Sullivan and Ikawa, (1973), Biochim. Biophys. Acts. 309, p. 11-22; Ikawa, (1982), Meth. in Enzymol. 89, Carbohydrate Metabolism Part D, 145-149) and is able to oxidize a wide variety of carbohydrates such as D-glucose, D-galactose, maltose, cellobiose, lactose, D-glucose-6-phosphate, D-mannose, 2-deoxy-D-glucose, 2-deoxy-D-galactose, D-fructose, D-glucuronic acid, D-xylose and the like. In the practice of the invention, these may be used irrespective of their origins.

Although depending on the form of preparation, frequency of use, treating time and titer of an oxidizing agent, the amount, in the composition, of the oxidase used in the invention usually ranges 0.0005 to 30% (wt% herein and whenever it appears hereinafter), preferably 0.005 to 20%, more preferably 0.005 to 15%, and most preferably 0.005 to 10%. If the amount is less than 0.0005%, a satisfactory effect may not be obtained in some case. Over 30%, the effect corresponding to an increase in the amount may not be attained.

The hair dye composition of the invention makes use of an oxidizing agent and an oxidation color as an component capable of color developing reaction. The oxidation color used in the invention is not critical with respect to its type and amount and may be selected from known ones. For instance, those described in Standards for Materials for Quasi-Drugs can be used in proper amounts, including 5-amino-o-cresol, o-aminophenol, m-aminophenol, p-aminophenol, 2,6-diaminopyridine, 5-(2-hydroxylethyl-amino)-2-methylphenol, N,N-bis(β-hydroxyl)-p-phenylenediamine·sulfate, N,N-bis(2-hydroxyethyl)-p-phenylenediamine, p-nitro-o-phenylenediamine, p-phenylenediamine, m-phenylenediamine, N-phenyl-p-phenylenediamine, resorcin, hydroquinone, 2-hydroxy-5-nitro-2',4'-diaminoazobenzene·sodium sulfate, toluene-2,5-diamine, 2-(2'-hydroxyethylamino)-5-aminotoluene, N,N-bis(β-hydroxyl)-p-phenylenediamine, N,N-bis(2-hydroxyethyl)-p-phenylenediamine·sulfate, 5-amino-o-cresol·sulfate, p-aminophenol·sulfate, o-chloro-p-phenylenediamine·sulfate, 2-(2'-hydroxyethylamino)-5-aminotoluene·sulfate, 4,4'-diaminodiphenylamine·sulfate, p-methylaminophenol· sulfate, p-phenylenediamine·sulfate, m-phenylenediamine· sulfate, toluene-2,5-diamine·sulfate, 2.4-diaminophenoxy-ethanol·hydrochloride, toluene-2,5-diamine·hydrochloride, m-phenylendiamine·hydrochloride, 2,4-diaminophenol·hydrochloride, 3,3'-iminodiphenyl, p-phenylenediamine·hydrochloride, N-phenyl-p-phenylendiamine·hydrochloride,N-phenyl-p-phenylenediamine·acetate, 1,5-dihyroxynaphthalene, toluene-3,4-diamine, p-methylaminophenol, N,N'-bis(4-aminophenyl)-2,5-diamino-1,4-quinonediimine, o-aminophenol sulfate, 2,4-diaminophenol·sulfate, m-aminophenol·sulfate and the like.

Direct dyes which are frequently used in combination with those oxidation colors may also be used as an oxidation color of the invention, including 2-amino-4-nitrophenol, 2-amio-5-nitrophenol, 1-amino-4-methylaminoanthraquinone, nitro-p-phenylenediamine·hydrochloride, 1,4-diaminoanthraquinone, nitro-p-phenylenediamine, picramic acid, sodium picramate, 2-amino-5-nitrophenol·sulfate, resorcinol, nitro-p-phenylenediamine·sulfate, p-nitro-o-phenylenediamine·sulfate. p-nitro-m-phenylenediamine·sulfate and the like.

Of these, p-phenylenediamine or salts thereof, toluene-2,5-diamine or salts thereof, p-aminophenol, 5-amino-o-cresol, m-aminophenol, p-nitro-o-phenylenediamine, 2,6-diaminopyridine, resorcinol, o-aminophenol, m-phenylenediamine and the like are preferably used. These oxidation colors may be used singly or in combination of two or more.

From a recent nature-oriented standpoint, a melanin precursor-like substance represented by the following general formula (2) may also be used as the oxidation dye of the invention 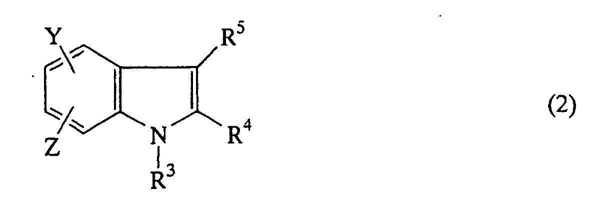 wherein Y represents a hydrogen atom, NH₂, OH, a linear or branched alkyl group, alkenyl group or alkoxyl group having 1 to 6 carbon atoms, and Z represents a hydrogen atom, OH or NH₂ provided that when Y is OH or a linear or branched alkyl group, alkenyl group or alkoxyl group having 1 to 6 carbon atoms, Y is at the 5, 6 or 7 position relative to the ring and is at the ortho position relative to Z, R³ and R⁵ may be the same or different and represents a hydrogen atom or a linear or branched alkyl group, alkenyl group or alkoxyl group having 1 to 6 carbon atoms, and R⁴ represents a hydrogen atom, or a linear or branched alkyl group, alkenyl group or alkoxyl group having 1 to 6 carbon atoms, or a carboxyl group.

The compounds represented by the above general formula (2) include, for example, 4,5-dihydroxylindole, 5,6-dihydroxyindole, 6,7- dihydroxyindole, N-methyl-5,6-dihydroxyindole, N-ethyl-5,6-dihydroxyindole, N-hexyl-5,6-dihydroxyindole, 2-methyl-5,6-dihydroxyindole, 3-methyl-5,6-dihydroxyindole, 4-hydroxyindole, 2,3-dimethyl-5,6-dihydroxyindole, 2-methyl-5-ethyl-6-hydroxyindole, 2-methyl-5-hydroxy-6-β-hydroxyethylindole, 4-hydroxypropylindole, 2-hydroxy-3-methoxyindole, 4-hydroxy-5-methoxyindole, 6-hydroxy-7-methoxyindole, 6-hydroxy-5-methoxyindole, 6-hydroxyindole, 5-hydroxyindole, 7-hydroxyindole, 7-aminoindole, 5-aminoindole, 4-aminoindole, 5,6-dihydroxyindole carboxylic acid, 1-methyl-5,6-dihydroxyindole and salts thereof, and the like.

A melanin precursor-like substance represented by the following general formula may also be used as the oxidation color of the invention 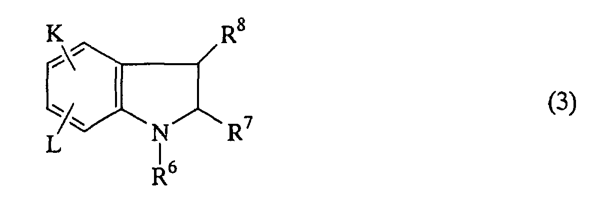 wherein K represents a hydrogen atom, NH₂, OH and a linear or branched alkyl group, alkenyl group or alkoxyl group having 1 to 6 carbon atoms, L represents a hydrogen atom, OH or NH₂ provided that when K is OH or a linear or branched alkyl group, alkenyl group or alkoxyl group having 1 to 6 carbon atoms, K is at the 5, 6 or 7 position relative to the ring and is at the ortho position relative to L, R⁶ and R⁸ may be the same or different and represent a hydrogen atom or a linear or branched alkyl group, alkenyl group or alkoxyl group having 1 to 6 carbon atoms, and R⁷ represents a hydrogen atom, a linear or branched alkyl group, alkenyl group or alkoxyl group having 1 to 6 carbon atoms, or a carboxyl group.

Specific examples of the compound represented by the above formula (3) include 4,5-dihydroxyindoline, 5,6-dihydroxyindoline, 6,7-dihydroxyindoline, N-methyl-5,6-dihydroxyindoline, N-ethyl-5,6-dihydroxyindoline, N-hexyl-5,6-dihydroxyindoline, 2-methyl-5,6-dihydroxyindoline, 3-methyl-5,6-dihydroxyindoline,4-hydroxyindoline, 2,3-dimethyl-5,6-dihydroxyindoline,2-methyl-5-ethyl-6-hydroxyindoline, 2-methyl-5-hydroxy-6-β-hydroxyethylindoline, 4-hydroxypropylindoline, 2-hydroxy-3-methoxyindoline, 4-hydroxy-5-methoxyindoline, 6-hydroxy-7-methoxyindoline, 6-hydroxy-5-methoxyindoline, 6-hydroxyindoline, 5-hydroxyindoline, 7-hydroxyindoline, 7-aminoindoline, 5-aminoindoline, 4-amioindoline, 5,6-dihydroxyindoline carboxylic acid, 1-methyl-5,6-dihydroxyindoline and salts thereof, and the like.

These compounds may be used singly or in appropriate combination of two or more from the standpoint of color matching.

The concentration in formulation of the oxidation color may be appropriately selected depending on the frequency in use and the type of preparation of a hair dye composition and is generally in the range of 0.01 to 10 %. preferably 0.1 to 5%, of the total composition.

The hair dye composition of the invention should be further formulated, aside from the above components, with a specific type of water-soluble high-molecular compound. The specific type of water-soluble high-molecular compound of the invention includes hydroxypropyl cellulose or carboxymethyl cellulose that is a cellulose-based water-soluble high-molecular compound, xanthan gum, gum guaiac, locust bean gum, gum arabic, tragacanth gum, karaya gum or gellan gum that is a gum-based water-soluble high-molecular compound, pectin, carrageenan, furcellaran, alginic acid and salts thereof, hyaluronic acid and salts thereof, or chondroitin sulfuric acid and salts thereof that are natural water-soluble high-molecular compounds, and ethylene oxide polymer, polyacrylic acid and salts thereof, acrylic copolymers and salts thereof, polyvinyl pyrrolidone, vinyl pyrrolidone copolymers, polyvinyl acetate, vinyl acetate copolymers, or carboxy vinyl polymer that is a synthetic water-soluble high-molecular compound. These may be used singly or in appropriate combination of two or more.

In the practice of the invention, ethylene oxide polymer should preferably have a degree of polymerization (n) of 50 or over, more preferably 100 or over. Polyacrylic acid includes, for example, a crosslinking polyacrylic acid, alkyl polyacrylates and the like. Examples of the polyacrylate include sodium polyacrylate, crosslinking sodium polyacrylate and the like. Moreover, examples of the acrylic copolymers and salts thereof include alkyl acrylate copolymers, alkyl acrylate copolymer emulsions, acrylic amide.styrene copolymers, alkyl acrylate·styrene copolymer emulsions, acrylic octylamide·acrylic ester copolymer, acrylic acid·acrylic amide·ethyl acrylate copolymer, a solution of a potassium salt of acrylic acid.acrylic amide· ethyl acrylate copolymer, acrylic octylamide·hydropxypropyl acrylate·butylaminoethyl methacrylate copolymer, and the like.

Examples of vinyl pyrrolidone copolymers and vinyl acetate copolymers include vinyl pyrrolidone·styrene copolymer emulsion, vinyl pyrrolidone·hexadecene copolymer, styrene·vinyl pyrrolidone copolymer, eicosene-vinyl pyrrolidone copolymer, vinyl acetate·vinyl pyrrolidone copolymer, vinyl acetate·crotonic acid copolymer, crotonic acid·vinyl acetate·vinyl neodecanoate copolymer, and the like.

More specifically, mention is made of "Junlon" made by Nihon Jiyunyaku KK, "Aron AP" made by Toagosei Chemical Ind. Co., Ltd., or the like as polyacrylic acid, "Matsumoto microspheres M series (M, M-100, M-101, M-305, M-306" made by Matsumoto Yushi-Seiyaku Co., Ltd. and the like as an alkyl polyacrylate, "Aronvis Series" and "Rheogic Series", both made by Nihon Jiyunyaku KK, "Aron AH Series" made by Toagosei Chemical Ind. Co., Ltd., as sodium polyacrylate, "Sanfresh ST-500 series" made by Sanyo Chemical Industries, Ltd. and the like as crosslinking sodium polyacrylate, "Luvimer 100P" made by BASF as an alkyl acrylate copolymer, "Ditozol 500SJ" made by Daito Kasei Kogyo Co., Ltd., "Yodosol GH series" made by Kanebo NSC, "Ditozol 5000AD" made by Daito Kasei Kogyo Co., Ltd., "LEOARL MS series" made by Lion Corp., and the like as an alkyl acrylate copolymer emulsion, "Yodosol GH52" made by Kanebo NSC and the like as an acrylic amide.styrene copolymer, "Yodosol GH41" made by Kanebo NSC, "Cevian-A46779" made by Daicel Chemical Industries, Ltd., "Ditozol SAP" made by Daito Kasei Kogyo Co., Ltd., and the like as an alkyl acrylate·styrene copolymer emulsion, "AMPHOMER V-42" made by Kanebo NSC, "Dermacryl series" made by Kanebo NSC and the like as an acrylic octylamide·acrylic acid ester copolymer, "Ultrahold strong 8" made by BASF and the like as an acrylic acid· acrylic amide·ethyl acrylate copolymer, "Ultrahold 8" made by BASF and the like as a potassium salt solution of acrylic acid·acrylic amide·ethyl acrylate copolymer, and "AMPHOMER 28-4910" made by Kanebo NSC and the like as an acrylic octylamide·hydroxypropyl acrylate·butylaminoethyl methacrylate copolymer.

In addition, mention is made of "PVP-K series" made by ISP. "Luviskol K resin" made by BASF, and the like as polyvinyl pyrrolidone, "ANTARA 430" made by ISP and the like as a vinyl pyrrolidone·styrene copolymer emulsion, "ANTARON V-216" made by ISP and the like as a vinyl pyrrolidone hexadecene copolymer, "ANTARA 430" made by ISP and the like as a vinyl pyrrolidone·styrene copolymer, "ANTARON V-220" made by BASF and the like as an eicosene·vinyl pyrrolidone copolymer, "Luviskol VA series" made by BASF, "NASUNA B" by Henkel, "PVP-VA series" made by ISP and the like as a vinyl acetate·vinyl pyrrolidone copolymer, "Resyn 28-1310" by Kanebo NSC, "Luviset CA66" made by BASF and the like as a vinyl acetate·crotonic acid copolymer, "Resyn 28-2930" by Kanebo NSC and the like as a crotonic acid·vinyl acetate· vinyl neodacanate copolymer, and "HIVISWAKO series" made by Wako Pure Chemical Industries, Ltd., as a carboxyvinyl polymer.

The amount of the water-soluble high-molecular compound in the hair dye composition of the invention is appropriately selected depending on the type thereof and is 0.05 to 30%, preferably 0.1 to 20%, of the total composition. If the amount is less than 0.05%, it may be difficult in some case to improve foaming properties when the hair dye composition of the invention is sprayed from an aerosol container. Over 30%, a difficulty may be involved, in some case, in improving the hair dyeing effect of the hair dye composition.

The hair dye composition of the invention becomes more excellent in foaming properties when a specific type of amido-type anionic surfactant represented by the following general formula (1) 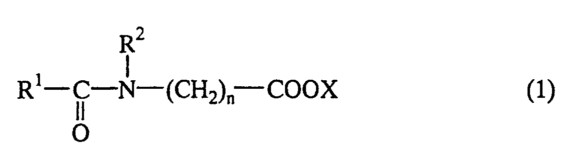 wherein R¹ a linear or branched alkyl group or alkenyl group having 8 to 30 carbon atoms, preferably 8 to 22 carbon atoms, R² represents a hydrogen atom or a linear or branched alkyl group or alkenyl group having 1 to 8 carbon atoms, preferably 1 to 6 carbon atoms, n is 2 to 7, preferably 2 to 5, X is a hydrogen atom, an ammonium group, a sodium atom, a potassium atom or an organic amine residue.

Specific examples of the anionic surfactant represented by the above general formula (1) include sodium N-lauroyl-N-methyl-β-alanine, triethanolamine N-lauroyl-N-methyl-β-alanine, sodium N-lauroyl-β-alanine, triethanolamine N-lauroyl-β-alanine and the like. These may be used singly or in appropriate combination of two or more.

The amount of the anionic surfactant in the hair dye composition of the invention may be appropriately selected depending on the type thereof, and is generally 0.01 to 5%, preferably 0.1 to 3%, of the total composition. If the amount is less than 0.01%, a difficulty may be involved, in some case, in satisfactorily improving foaming properties. Over 5%, not only stickiness may develop with poor usability, but also the activity of an oxidase existing in the composition may be impeded.

If necessary, the hair dye composition of the invention may further comprise, within ranges not impeding the effect of the invention, a pH adjusting agent for adjusting the composition to a given pH as will be described hereinafter, and surfactants other than those set out hereinabove, a thickening agent such as hydroxyethyl cellulose, a perfume, a preservative, a UV absorber, an antioxidant, a bactericide, a pearling agent and the like. Mention is made, as the pH adjusting agent, of acids including organic acids such as glycollic acid, lactic acid, tartaric acid, acetic acid, citric acid, malic acid, succinic acid and the like, and inorganic acids such as phosphoric acid, hydrochloric acid and the like, and alkalis including organic alkalis such as monoethanolamine, triethanolamine and the like and inorganic alkalis such as sodium hydroxide, potassium hydroxide and the like, and as a surfactant, anionic surfactants such as α-olefinsulfonates, alkanesulfonates, aliphatic acid alkyl ether carboxylates, N-acylamino acid salts, C₁₂ to C₁₈ saturated and unsaturated fatty acid acyl glutamic acid esters and the like, amphoteric surfactants such as alkyl betaines, alkylamido betaines, hydroxysulfo betaines, and the like, cationic surfactants such as mono or dialkyl quaternary ammonium salts, and nonionic surfactants such as polyoxyethylene alkyl ethers, fatty acid alkylolamides and the like. In the practice of the invention, these surfactants may be formulated so as to control the composition at a given viscosity by combination with the afore-indicated water-soluble high-molecular compound as will be described hereinafter.

In order to improve a feel to the touch of hair, silicone derivatives such as dimethylpolysiloxane, amino-modified silicones, polyether-modified silicones and the like may be further formulated.

The hair dye composition of the invention should preferably have a pH of 5 to 8.5, more preferably 6 to 7.5. If the pH is less than 5, the stability of the composition may, in some case, be much worsened. Over 8.5, dyeing properties may lower. In the invention, the pH of the composition is adjusted by use of such a pH adjusting agent as mentioned hereinbefore.

It is favorable that the hair dye composition of the invention should have a viscosity (25°C) of 5 to 30,000 mPa· s, preferably 10 to 20,000 mPa·s, more preferably 100 to 10,000 mPa·s and most preferably 1,000 to 5,000 mPa·s. If the viscosity is less than 5 mPa·s, the dripping of liquid may take place upon application to hair. On the other hand, when the viscosity exceeds 30,000 mPa·s, dyeing properties may lower.

In the practice of the invention, the viscosity can be measured according to a viscosity measuring method described in the general testing methods of the Japanese Pharmacopoeia. Where the viscosity of the hair dye composition of the invention is controlled, the viscosity of the hair dye composition prior to filling in an aerosol container described hereinafter is so controlled as to be within such a range as defined above.

The hair dye composition of the invention is prepared by dissolving an oxidation color, an oxidase and, if necessary, a specific type of amide-type anionic surfactant as specified before and arbitrary additives in water, adjusting the viscosity and pH of the resulting solution to provide a stock solution, filling the stock solution in an aerosol container, and filling an aerosol propellant in the container to obtain a one-pack aerosol type hair dye composition. The formulation in the invention should preferably be carried out in vacuum or in an atmosphere of an inert gas such as nitrogen gas, carbon dioxide gas, a rare gas or the like. A specific manner of providing such an atmosphere may be any of known ones and is not critical. For instance, a formulation facility and environment may be evacuated to vacuum or may be substituted with nitrogen gas, or a method of substituting with an inert gas after evacuation to vacuum may be mentioned. Where such a formulation method is adopted, filling of an aerosol propellant may be effected in air and is preferably carried out in an atmosphere of a vacuum or an inert gas such as nitrogen gas, carbon dioxide gas, a rare gas or the like. A specific manner of creating such an atmosphere may be any one and is not critical.

The aerosol propellant used for the hair dye composition of the invention as a one-pack aerosol type oxidation hair dye composition includes a liquefied petroleum gas (LPG), dimethyl ether (DME), nitrogen gas, carbon dioxide gas, nitrous oxide gas, rare gas, flon 11, flon 12, flon 114 and the like, used singly or in combination of two or more. Of these, LPG is preferred from the standpoint of foam forming properties after spraying.

In the practice of the invention, the mixing ratio (by weight) of the stock solution and the aerosol propellant is such that stock solution:aerosol propellant = 95:5 to 85:15, preferably 94:6 to 90:10, and the gas pressure of the aerosol propellant filled in an aerosol container should be 2 kg/cm² or over, preferably 2 to 5 kg/cm² and more preferably 3 to 4.5 kg/cm², under which when the hair dye composition of the invention is sprayed from the aerosol container, the spraying properties and foam forming properties after spraying becomes more excellent.

It will be noted that any known aerosol container may be used for the aerosol container and may include not only a direct spraying type as placed in an ordinary aluminium can, a tinplate can, a PET container or the like, but also another type of double container such as a piston type, a back-in type, an EXXEL type or the like.

The invention is particularly described by way of examples and comparative examples, and the invention should not be construed as limiting to the examples. It will be noted that % in the examples is by weight.

### [Examples, Comparative Examples]

Stock solutions for one-pack aerosol type hair dye compositions were, respectively, prepared according to an ordinary method using formulations indicated in Table 1, and the stock solution were, respectively, packed in an aerosol test bottle (made by Tokyo Polymer Co., Ltd.) and clinched in vacuum, followed by filling 4.0 kg of LPG serving as an aerosol propellant so that stock solution:gas = 90:10 (by weight ratio) to obtain aerosol-type hair dye compositions of examples and comparative examples.

The thus obtained one-pack aerosol type hair dye compositions were subjected to evaluation of the following tests. The results are also shown in Table 1.

### Dyeing property (ΔE):

About 10 g of dried white hair of goat was shampooed, and water was drained (the weight of hair bundle after the draining of water was at 17g). 3g of each one-pack aerosol type hair dye composition stored one month at 45°C immediately after preparation was quickly, uniformly applied to the hair. After allowing to stand at 30°C over certain period of time, the hair was rinsed. After drying, the hair was shampooed and air dried to evaluate the dyeing property of the goat hair.

The dyeing property (ΔE) was evaluated by subjecting the dyed hair bundle to measurement of values of L, a and b by use of a differential colorimeter (SE2000. made by Nippon Denshoku Industries Co., Ltd.), from which a color difference (ΔE) from undyed hair was determined to assess the dyeing property. It will be noted that a greater value of ΔE indicates a better dyeing property.

### <Drippiness of liquid>

A dried human hair bundle (30 cm, 10 g) was shampooed and dried. This hair bundle was suspended, to which each aerosol-type hair dye was sprayed to provide an evaluation sample, and 10 g of each composition was applied to the hair bundle by use of a brush. After the application, the hair bundle was allowed to stand for 30 minutes, during which presence or absence of the dripping of the liquid was visually observed.

### <Storage stability>

The presence or absence of an insoluble matter and a coagulum in the respective aerosol-type hair dye compositions and a color change thereof were, respectively, visually observed from outside containers immediately after the preparation of the compositions (filled in the aerosol container), after storage at room temperature over 6 months, and after storage at 45°C over 1 month, followed by evaluation according to the following evaluation standards.

The evaluation was made at n = 5, and an evaluation point at which the evaluation was most concentrated was provided as a result of the evaluation.

### Evaluation standards

ⓞ: not recognized at all
○: recognized when carefully observed
Δ: clearly recognized
×: observed in so many a number as not to stand use.

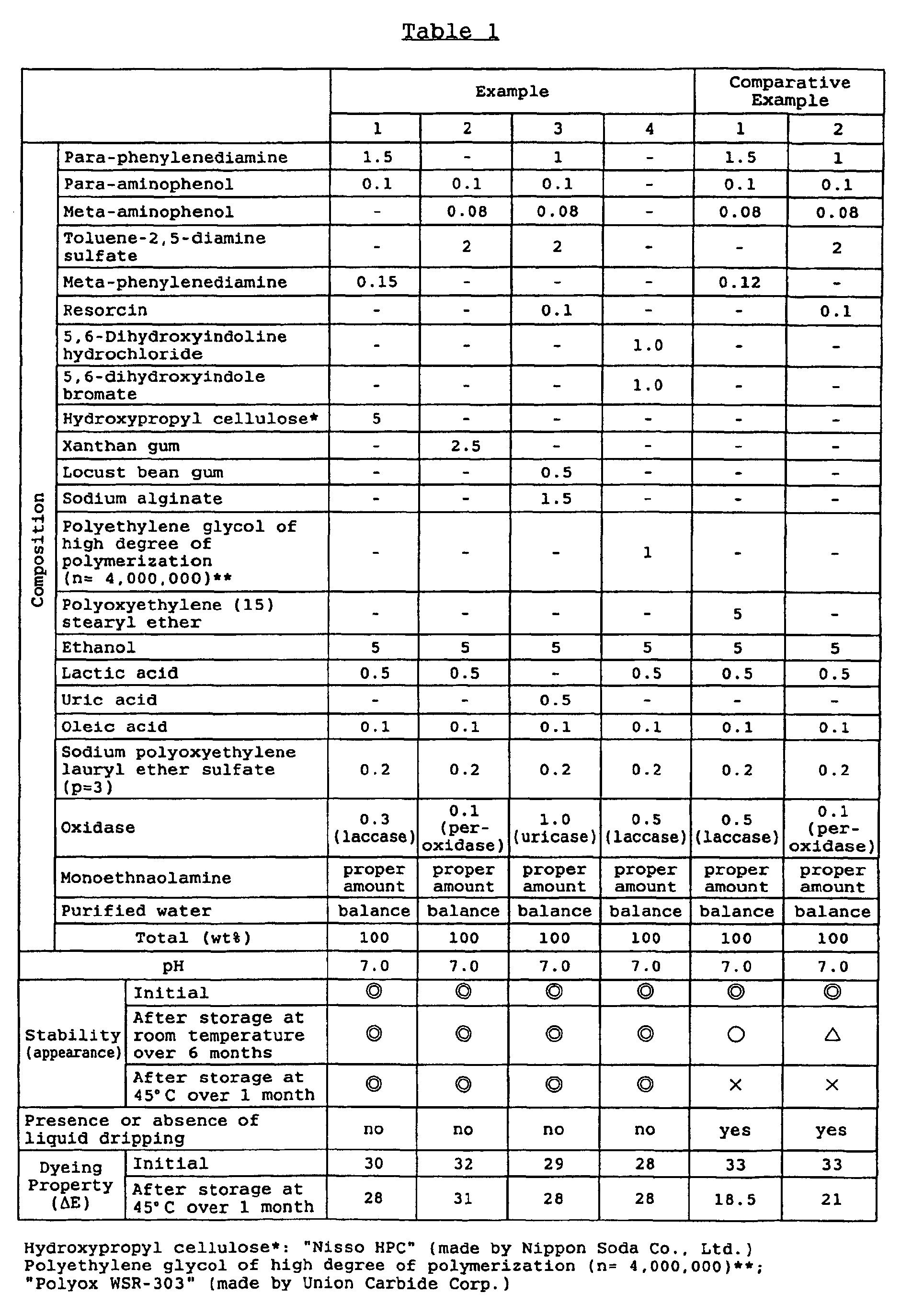

According to the results of Table 1, it is recognized that the hair dye compositions of the invention exhibits excellent stability properties by formulating a specific type of water-soluble high-molecular compound.

### [Experimental Example]

Next, the stock solution of the hair dye composition of Example 1 was used to evaluate spraying properties of foams by changing the ratio by weight between the stock solution and an aerosol propellant and a gas pressure of the aerosol propellant as shown in Table 2. The foam-forming property immediately after spraying was evaluated according to the following evaluation standards. The results are also shown in Table 2.
Evaluation standards of foam-forming property immediately after spraying
ⓞ: Steady, creamy foams are sprayed.
○: Steady foams are sprayed.
Δ: Foams are not formed immediately after spraying and subsequently foaming takes place.
×: The stock solution is scattered without formation of foams.

**Table 2**

| | Gas pressure | Stock solution:aerosol propellant (ratio by weight) | | | | | |
|---|---|---|---|---|---|---|---|
| | | 95:5 | 93:7 | 90:10 | 85:15 | 97.5:2.5 | 80:20 |
| Spraying property of foams | 3.0 kg/cm2 | good | good | good | good | bad | bad |
| Foam-forming property immediately after spraying | | ⓞ | ⓞ | ⓞ | ⓞ | × | Δ |
| Spraying property of foams | 4.0 kg/cm² | good | good | good | good | bad | bad |
| Foam-forming property immediately after spraying | | ⓞ | ⓞ | ⓞ | ⓞ | × | Δ |
| Spraying property of foams | 4.5 kg/cm² | good | good | good | good | bad | bad |
| Foam-forming property immediately after spraying | | ⓞ | ⓞ | ⓞ | ⓞ | Δ | Δ |
| Spraying property of foams | 1.5 kg/cm² | good | good | good | good | bad | bad |
| Foam-forming property immediately after spraying | | × | Δ | Δ | Δ | × | Δ |

### [Examples, Comparative Examples]

The respective components were mixed by an ordinary method using the formulations indicated in Tables 3 and 4, followed by adjusting the pH to values indicated in Tables 3 and 4 to prepare stock solutions for one-pack type hair dye composition. The respective stock solutions were filled in a transparent pressure bottle and clinched in vacuum, after which 4.0 kg of LPG serving as an aerosol propellant was filled so that stock solution:gas = 95:5 (ratio by weight) to obtain aerosol-type hair dye compositions of examples and comparative examples. It will be noted that the viscosities indicated in Tables 3 and 4 are those measured by means of a Brookfield type viscometer immediately after preparation thereof.

The thus obtained aerosol-type hair dye compositions were, respectively, evaluated with respect to the liquid dripping, storage stability and dyeing property in the same manner as in Example 1. The results are also shown in Tables 3 and 4.

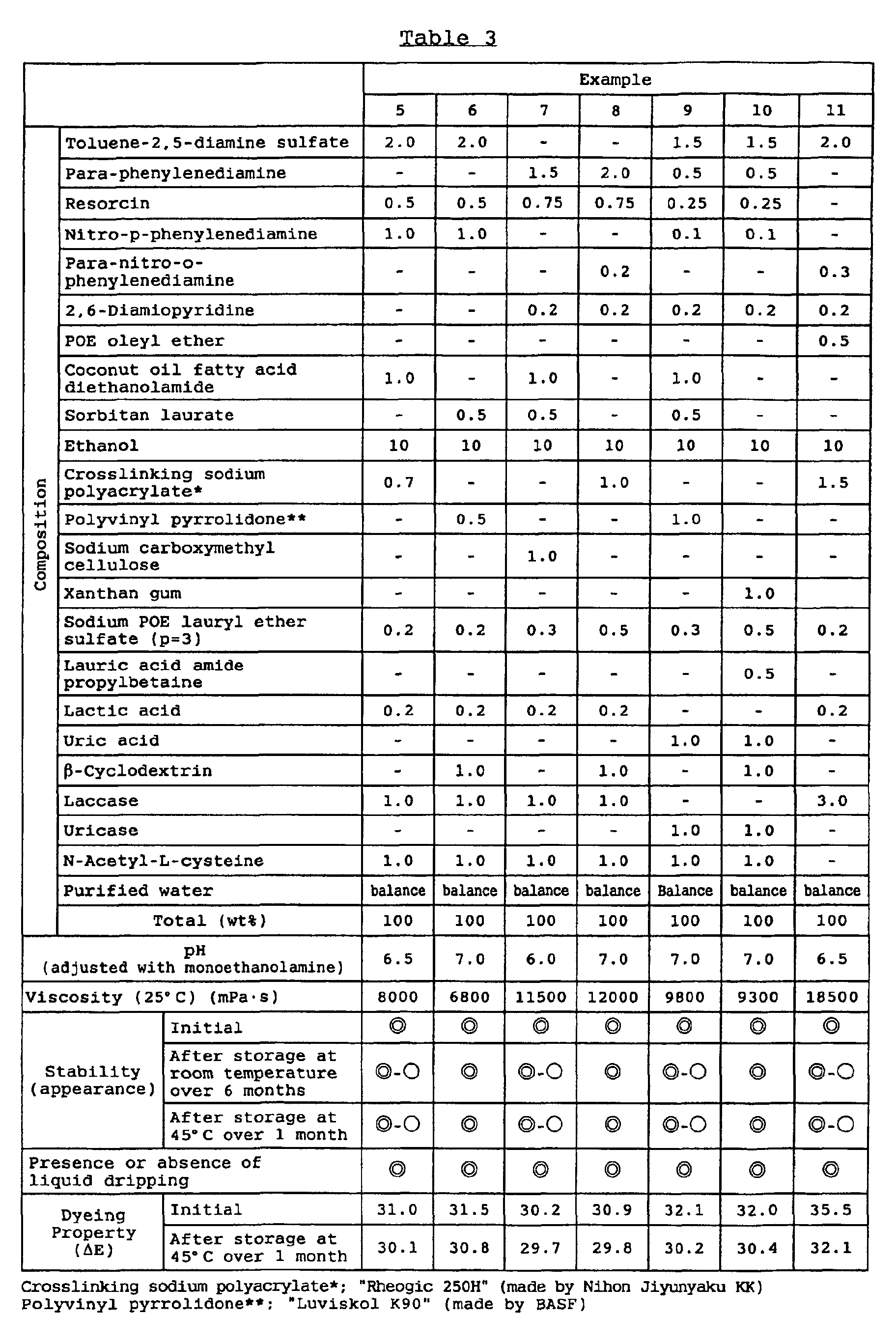

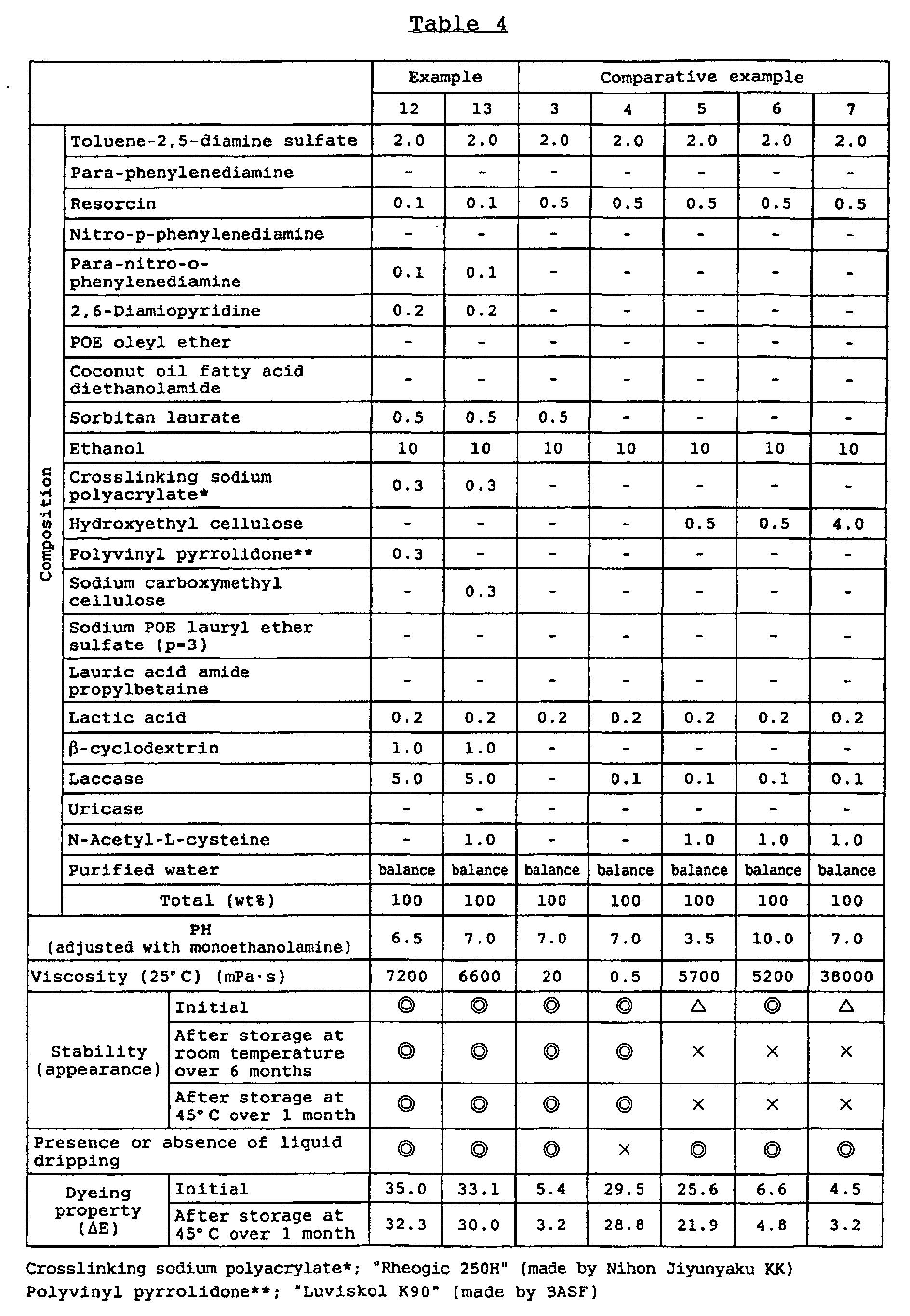

More specific formulations of the hair dye composition of the invention are indicated by way of examples. Using the formulations indicated below, stock solutions of one-pack aerosol type hair dye composition were prepared by a usual method, and each stock solution was filled in an aerosol can, followed by clinching in vacuum and further filling 4.0 kg of LPG serving as an aerosol propellant in such a way that stock solution:gas = 90:10 (ratio by weight), thereby obtaining one-pack aerosol type hair dye compositions of Example 14 to 17. 1 g of the thus obtained one-pack aerosol type hair dye composition was sprayed and applied onto 1.0 g of white hair and allowed to stand for 20 minutes, revealing that no liquid dripping was found at a glance for any of the compositions. Thereafter, the hair was washed with water and shampooed. The thus treated white hair was dried with a dryer, revealing that all of the compositions could impart a good feel to the touch to the hair and that the white hair was dyed dark grayish black. The color tone was the same as that attained immediately after preparation.

| [Example 14] | |
|---|---|
| Formulation components | Amount (wt%) |
| Para-phenylenediamine | 1 |
| Para-aminophenol | 0.1 |
| Meta-aminophenol | 0.2 |
| Hydroxypropyl cellulose* | 2 |
| Xanthan gum | 0.5 |
| Laccase | 5 |
| Monoethanolamine | proper amount |
| Purified water | balance |
| Total | 100 |
| pH 6.5 | |

| | |
|---|---|
| Hydroxypropyl cellulose*; "Nisso HPC" (made by Nippon Soda Co., Ltd.) | |

| [Example 15] | |
|---|---|
| Formulation components | Amount (wt%) |
| p-Phenylenediamine | 0.5 |
| Toluene-2,5-diamine sulfate | 1 |
| Decaglyceryl monostearate | 3 |
| Cetostearyl alcohol | 0.5 |
| Stearic acid | 0.8 |
| Laccase | 15 |
| Xanthan gum | 1 |
| Carboxymethyl cellulose | 1 |
| Sodium hydroxide | proper amount |
| Purified water | balance |
| Total | 100 |
| pH 7.0 | |

| [Example 16] | |
|---|---|
| Formulation components | Amount (wt%) |
| p-Phenylenediamine | 2 |
| Toluene-2,5-diamine sulfate | 1 |
| Meta-phenylenediamine | 0.1 |
| Meta-aminophenol | 0.8 |
| Laccase | 2.5 |
| Highly polymerized polyethylene glycol(n=4,000,000)* | 0.3 |
| Polyvinyl alcohol | 0.1 |
| POE(10) cetyl ether | 8 |
| Stearyl alcohol | 2.5 |
| Oleyl alcohol | 5 |
| Behenyl alcohol | 2 |
| Cetyl alcohol | 2 |
| Stearyltrimethylammonium chloride | 1 |
| Glycerine | 2 |
| Triethanolamine | proper amount |
| Purified water | balance |
| Total | 100 |
| pH 6.5 | |

| | |
|---|---|
| Highly polymerized polyethylene glycol (n=4,000,000)*; "Polyox WSR-303" (made by Union Carbide Corp.) | |

| [Example 17] | |
|---|---|
| Formulation components | Amount (wt%) |
| p-Phenylenediamine | 2 |
| Toluene-2,5-diamine sulfate | 1 |
| Meta-phenylenediamine | 0.1 |
| Meta-aminophenol | 0.8 |
| Laccase | 12 |
| Gum guaiac | 0.5 |
| Carboxyvinyl polymer* | 0.5 |
| Amisoft (made by Ajinomoto Co., Inc.) | 1 |
| Glycerine | 2 |
| Triethanolamine | proper amount |
| purified water | balance |
| Total | 100 |
| pH 7.0 | |

| | |
|---|---|
| Carboxyvinyl polymer*; "Carbopol 1342" (made by B. F. Goodrich) | |

More specific formulations of the hair dye composition of the invention wherein pH and viscosity are, respectively, controlled are shown as Examples 18 to 20.

| [Example 18] | |
|---|---|
| Formulation components | Amount (wt%) |
| 5,6-Dihydroxyindoline bromate | 1.0 |
| 5,6-Dihydroxyindole hydrochloride | 1.0 |
| N-Ethyl-5,6-dihydroxyindole hydrochloride | 0.05 |
| Linolic acid | 0.2 |
| Oleyl alcohol | 0.2 |
| β-cyclodextrin | 1.0 |
| Laccase | 5.0 |
| Hydroxypropyl cellulose | 0.2 |
| Sodium coconut oil fatty acid acyl-glutamate | 1.0 |
| N-Acetyl-L-cysteine | 0.5 |
| Thiourea | 0.3 |
| Ethanol | 10.0 |
| Lactic acid | 0.2 |
| Purified water | balance |
| Total | 100 |
| (pH was adjusted to 6.8 by means of monoethanolamine.) Hydroxypropyl cellulose*; "Nisso HPC" (made by Nippon Soda Co., Ltd.) | |

A stock solution of a one-pack aerosol type hair dye composition was prepared according to an ordinary method using the above formulation, and the stock solution was filled in an aerosol can, followed by clinching in vacuum and further filling 4.0 kg of LPG serving as an aerosol propellant in such a way that stock solution:gas = 95:5 (ratio by weight), thereby obtaining an aerosol-type hair dye composition. 1 g of this hair dye composition was applied onto 1 g of white hair and allowed to stand for 20 minutes, followed by washing with water and shampooing. The resulting white hair was dried with a dryer, revealing that the white hair was dyed black, and the color tone underwent no change from that attained immediately after preparation. During the course between the application and the washing with water, no liquid dripping was found. It will be noted that the viscosity of the stock solution (25°C) was at 3800 mPa·s.

| [Example 19] | |
|---|---|
| Formulation components | Amount (wt%) |
| 5,6-Dihydroxyindoline bromate | 1.0 |
| 5,6-Dihydroxyindole hydrochloride | 1.0 |
| N-Methyl-5,6-dihydroxyindine bromate | 0.5 |
| N-Methyl-5,6-dihydroxyindole hydrochloride | 0.5 |
| 5-Aminoindole hydrochloride | 0.25 |
| 2,3-Dimethyl-5,6-dihydroxyindoline bromate | 0.25 |
| Oleic acid | 0.5 |
| Linolic acid | 0.5 |
| Laccase | 5.0 |
| Xanthan gum | 1.0 |
| N-Acetyl-L-cysteine | 1.0 |
| POE lauryl ether | 1.0 |
| Lactic acid | 0.2 |
| Purified water | balance |
| Total | 100 |
| (pH was adjusted to 7.0 by means of monoethanolamine.) | |

A stock solution of a one-pack aerosol type hair dye composition was prepared according to an ordinary method using the above formulation, and the stock solution was filled in an aerosol can, followed by clinching in vacuum and further filling 4.0 kg of LPG serving as an aerosol propellant in such a way that stock solution:gas = 95:5 (ratio by weight), thereby obtaining an aerosol-type hair dye composition. 1 g of this hair dye composition was applied onto 1 g of white hair and allowed to stand for 20 minutes, followed by washing with water and shampooing. The resulting white hair was dried with a dryer, revealing that the white hair was dyed grayish black, and the color tone underwent no change from that attained immediately after preparation. During the course between the application and the washing with water, no liquid dripping was found. It will be noted that the viscosity of the stock solution (25°C) was at 11500 mPa·s.

| [Example 20] | |
|---|---|
| Formulation components | Amount (wt%) |
| 5,6-Dihydroxyindoline bromate | 1.0 |
| 5,6-Dihydroxyindole hydrochloride | 1.0 |
| N-Ethyl-5,6-dihydroxyindole hydrochloride | 0.05 |
| Sorbitan oleate | 0.2 |
| β-Cyclodextrin | 1.0 |
| Laccase | 3.0 |
| N-Acetyl-L-cysteine | 1.0 |
| Sodium polyacrylate* | 0.5 |
| Ethanol | 10.0 |
| glycollic acid | 0.2 |
| Purified water | balance |
| Total | 100 |
| (pH was adjusted to 6.8 by means of monoethanolamine.) | |

| | |
|---|---|
| Sodium polyacrylate*; "Aronvis S" (Nihon Jiyunyaku KK) | |

A stock solution of a one-pack aerosol type hair dye composition was prepared according to an ordinary method using the above formulation, and the stock solution was filled in an aerosol can, followed by clinching in vacuum and further filling 4.0 kg of LPG serving as an aerosol propellant in such a way that stock solution:gas = 95:5 (ratio by weight), thereby obtaining an aerosol-type hair dye composition. 1 g of this hair dye composition was applied onto 1 g of white hair and allowed to stand for 20 minutes, followed by washing with water and shampooing. The resulting white hair was dried with a dryer, revealing that the white hair was dyed black, and the color tone underwent no change from that attained immediately after preparation. During the course between the application and the washing with water, no liquid dripping was found. It will be noted that the viscosity of the stock solution (25°C) was at 5300 mPa·s.

### [Examples]

Stock solutions for one-pack aerosol type hair dye composition were, respectively, prepared according to an ordinary method using formulations indicated in Table 5. Each stock solution was filled in an aerosol test bottle (made by Tokyo Polymer Co., Ltd.), followed by clinching in vacuum and further filling 4.0 kg of LPG or the like so that stock solution:gas = 90:10 (ratio by weight) to obtain aerosol-type hair dye compositions of examples and comparative examples. The resulting one-pack aerosol type hair dye compositions were evaluated with respect to dyeing property (immediately after formulation) in the same manner as in Example 1 and were also tested and evaluated as set out below.

### Foam forming property:

A composition filled in an aerosol container was sprayed and the state of formed foams was visually observed to judge the property based on the following evaluation standards.

### Evaluation standards

ⓞ: Fine, steady foams are sprayed.
○: Steady foams are sprayed.
Δ: Foams are not sprayed, after which foaming takes place.
×: A liquid is sprayed without foaming.

### Shape retention of foams:

The state of foam breakage after spraying a composition filled in an aerosol container was visually observed and judged based on the following evaluation standards.

### Evaluation standards

ⓞ: After foaming, good form retention is shown without breakage.
○: Foams are retained without breakage of foams immediately after foaming.
Δ: Foams commence to break immediately after foaming.
×: A sprayed one is liquid and does not function as foams.

**Table 5**

| Formulation components (100 wt% in total) | Example | | | | | | | | | | |
|---|---|---|---|---|---|---|---|---|---|---|---|
| | 21 | 22 | 23 | 24 | 25 | 26 | 27 | 28 | 29 | 30 | 31 |
| p-Phenylenediamine | | 1.5 | 1.5 | 1 | | | | | | | |
| p-Aminophenol | 0.1 | 0.1 | 0.1 | 0.1 | | | 0.1 | 0.1 | | 0.1 | |
| m-Aminophenol | 0.08 | | | 0.08 | | | | 0.3 | 0.1 | 0.08 | |
| Toluene-2,5-diamine sulfate | 2 | | | 2 | | | 1.5 | 1 | | 2 | |
| 2,4-Diaminophenoxyethanol hydrochloride | | 0.15 | 0.15 | | | | | 0.1 | | | |
| Resorcin | | | | 0.1 | | | | | | | |
| 5,6-Dihydroxyindoline hydrochloride | | | | | 1.0 | 2 | | | 1.5 | | 1.0 |
| 5,6-Dihydroxyindole bromate | | | | | 1.0 | | | | | | 1.0 |
| Ethanol | 5 | 5 | 5 | 5 | 5 | 5 | 5 | 5 | 5 | 5 | 5 |
| Lactic acid | 0.5 | 0.5 | 0.5 | 0.5 | 0.5 | 0.5 | 0.5 | 0.5 | 0.5 | 0.5 | 0.5 |
| Oleic acid | 0.1 | 0.1 | 0.1 | 0.1 | 0.1 | 0.1 | 0.1 | 0.1 | 0.1 | 0.1 | 0.1 |
| N-lauroyl-N-methyl-β-alanine·TEA | 0.3 | | | 1 | | 3 | | 1.5 | | | |
| N-lauroyl-N-methyl-β-alanine·sodium | | 0.5 | | | 1.5 | | 2.5 | 0.5 | | | |
| N-lauroyl-β-alanine TEA | | | 2 | 1 | 0.5 | | 0.05 | | | | |
| N-lauroyl-sarcosine TEA | | | | | | | | | 2 | 0.5 | 1.5 |
| N-lauroyl-sarcosine sodium | | | | | | | | | | | 0.5 |
| Oxidase | 0.03 (laccase) | 0.1 (peroxidase) | 0.01 (catecoloxidase) | 0.05 (laccase) | 0.05 (laccase) | 0.03 (laccase) | 0.1 (peroxidase) | 0.01 (catecoloxidase) | 0.05 (laccase) | 0.05 (laccase) | 0.1 (uricse) |
| Xanthan gum | 2 | | | | | 1.3 | 0.3 | | | 0.3 | |
| Crosslinking sodium polyacrylate* | | 0.2 | 1.5 | | | 0.5 | 0.2 | | 0.75 | | |
| Sodium polyacrylate** | | | | 2 | | | | 0.8 | | 1.5 | |
| Polyvinyl pyrrolidone*** | | | 0.5 | | 1 | | 0.5 | 0.3 | | | 2 |
| Monoethanolamine | Proper Amount | Proper Amount | Proper Amount | Proper Amount | Proper Amount | Proper Amount | Proper Amount | Proper Amount | Proper Amount | Proper Amount | Proper Amount |
| Aerosol propellant | LPG | LPG | LPG | DME | DME | LPG | CO₂ | LPG | N₂ | LPG | Isopen -tane |
| Purified water | balance | balance | balance | balance | balance | balance | balance | balance | balance | balance | balance |
| PH | 7.0 | 7.0 | 7.0 | 7.0 | 7.0 | 7.0 | 7.0 | 7.0 | 7.0 | 7.0 | 7.0 |
| Dyeing property (ΔE) of product obtained immediately after formulation | 26 | 28 | 31 | 30 | 32 | 30 | 28 | 34 | 30 | 31 | 27 |
| Foam forming property | ⓞ | ⓞ | ⓞ | ⓞ | ⓞ | ⓞ | ⓞ | ⓞ | ○ | ○ | ○ |
| Foam retention | ⓞ | ⓞ | ⓞ | ⓞ | ⓞ | ⓞ | ⓞ | ⓞ | ○ | ○ | ○ |

| | | | | | | | | | | | |
|---|---|---|---|---|---|---|---|---|---|---|---|
| Crosslinking sodium polyacrylate*; "Rheogic 250H" (made by Nihon Jiyunyaku KK) | | | | | | | | | | | |
| Sodium polyacrylate**; "Aronvis S" (made by Nihon Jiyunyaku KK) | | | | | | | | | | | |
| Polyvinyl pyrrolidone***; "Luviskol K90 (made by BASF) | | | | | | | | | | | |

According to the invention, a one-pack aerosol type oxidation hair dye, which can save the trouble of mixing a first agent and a second agent whenever used and is excellent in utility, is improved in foaming property when sprayed from a container and is further improved in usability such as of preventing liquid dripping on use. Moreover, there can be obtained a one-pack aerosol type hair dye composition that has good stability with time, shows an excellent hair-dyeing effect after storage over a long time and ensures excellent foaming properties.

## Claims

1. A one-pack aerosol type hair dye composition which comprises an oxidation color and an oxidase, **characterized in that** at least one water-soluble high-molecular compound selected from hydroxypropyl cellulose, carboxymethyl cellulose, xanthan gum, gum guaiac, locust bean gum, gum arabic, tragacanth gum, karaya gum, gellan gum, pectin, carrageenan, furcellaran, alginic acid and salts thereof, hyaluronic acid and salts thereof, chondroitin sulfuric acid and salts thereof, ethylene oxide polymer, polyacrylic acid and salts thereof, acrylic copolymers and salts thereof, polyvinyl pyrrolidone, vinyl pyrrolidone copolymers, polyvinyl acetate, vinyl acetate copolymers, carboxy vinyl polymer is formulated.

2. The hair dye composition according to Claim 1, wherein said composition has a pH of 5 to 8.5 and a viscosity of 5 to 30,000 mPa·s at 25°C.

3. The hair dye composition according to Claim 1 or 2, further comprising an amido-type surfactant represented by the following general formula (1) and having a carboxyl group or a salt thereof at a terminal thereof 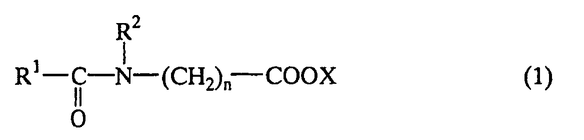 (wherein R¹ represents a linear or branched alkyl group or alkenyl group having 8 to 30 carbon atoms, R² represents a hydrogen atom or a linear or branched alkyl group or alkenyl group having 1 to 8 carbon atoms, n is 2 to 7, and X represents a hydrogen atom, an ammonium group, a sodium atom, a potassium atom or an organic amine residue).

4. The hair dye composition according to Claim 1, 2 or 3, wherein a ratio by weight of a stock solution for said one-pack aerosol type hair dye composition and an aerosol propellant is such that stock solution: aerosol propellant = 95:5 to 85:15 and a gas pressure of said aerosol propellant is at 2kg/cm² or over.
